# FASCICULE DE BREVET EUROPEEN

(11) **EP 0 651 801 B1**
(45) Date de publication et mention de la délivrance du brevet: **05.11.2003**
(21) Numéro de dépôt: 93913199.1
(22) Date de dépôt: 29.06.1993
(51) Int. Cl.: C12N 15/12, C07H 21/00, C12Q 1/68, G01N 33/50, A61K 31/00

(54) **POLYPEPTIDES AYANT UNE ACTIVITE DE RECEPTEUR SEROTONINERGIQUE (5HT5A), ACIDES NUCLEIQUES CODANT POUR CES POLYPEPTIDES ET UTILISATIONS**
POLYPEPTIDE MIT SEROTONINERGER (5HT5A) REZEPTOR-AKTIVITÄT, FÜR SIE KODIERENDE NUKLEINSÄUREN UND IHRE VERWENDUNG
POLYPEPTIDES HAVING SEROTONINERGIQUE ACTIVITY (5HT5A), NUCLEIC ACIDS CODING FOR THESE POLYPEPTIDES AND USES THEREOF

(30) Priorité: 01.07.1992 FR 9208081
(43) Date de publication de la demande: 10.05.1995
(73) Titulaire: INSTITUT NATIONAL DE LA SANTE ET DE LA RECHERCHE MEDICALE (INSERM), 75654 Paris Cédex 13 (FR)
(72) Inventeur: AMLAIKY, Nourdine, F-67000 Strasbourg (FR); BOSCHERT, Ursula, F-67000 Strasbourg (FR); HEN, René, F-67000 Strasbourg (FR); PLASSAT, Jean-Luc, F-67100 Strasbourg (FR)
(74) Mandataire: Gillard, Marie-Louise
(86) Numéro de dépôt international: FR9300650
(87) Numéro de publication internationale: WO94001555

(56) Documents cités:
- WO-A-91/17174
- DE-A- 4 041 464
- PROCEEDINGS OF THE NATIONAL ACADEMY OF SCIENCES OF USA. vol. 89, no. 12, 15 Juin 1992, WASHINGTON US pages 5517 - 5521 McAllister G;Charlesworth A;Snodin C;Beer MS;Noble AJ;Middlemiss DN;Iversen LL;Whiting P; 'Molecular cloning of a serotonin receptor from human brain (5HT1E): a fifth 5HT1-like subtype.'
- EMBO JOURNAL. vol. 11, no. 13, Décembre 1992, EYNSHAM, OXFORD GB pages 4779 - 4786 PLASSAT, J.L. ET AL.; 'The mouse 5HT5 receptor reveals a remarkable heterogeneity within the 5HT1D receptor family'
- PROCEEDINGS OF THE NATIONAL ACADEMY OF SCIENCES OF USA. vol. 90, Avril 1993, WASHINGTON US pages 3452 - 3456 ERLANDER M.G. ET AL.; 'Two members of a distinct subfamily of 5-hydroxytryptamine receptors differentially expressed in rat brain'

## Description

La présente invention concerne de nouveaux polypeptides et le matériel génétique permettant leur expression. Plus particulièrement, elle concerne de nouveaux polypeptides ayant une activité de récepteur sérotoninergique.

La sérotonine est un neuromodulateur capable d'induire et de moduler une grande variété de comportements tels que le sommeil, l'appétit, la locomotion, l'activité sexuelle ou encore la contraction vasculaire. Il est admis que l'activité de la sérotonine est médiée par son interaction avec des récepteurs, désignés récepteurs sérotoninergiques ou récepteurs 5-HT (pour 5-hydroxytryptamine). Des études de biologie moléculaire ainsi que des études pharmacologiques ont révélé qu'il existait un grand nombre de sous-types de récepteurs 5-HT. Les récepteurs 5-HT qui ont été décrits jusqu'à aujourd'hui appartiennent soit à la famille des récepteurs liés à des canaux ioniques (récepteurs 5-HT3), soit à la famille des récepteurs qui interagissent avec des protéines G et qui possèdent sept domaines transmembranaires. Par ailleurs, l'analyse des séquences d'acides aminés a montré que les récepteurs 5-HT interagissant avec des protéines G peuvent être sous-divisés en deux groupes distincts : Les récepteurs 5HT1, comprenant les sous-types mammifères 5HT1A, 5HT1B et 5HT1D ainsi que trois récepteurs 5HT de drosophile; et les récepteurs 5HT2 comprenant les sous-types 5HT2 et 5HT1C.

Ces récepteurs ne sont sans doute pas les seuls récepteurs 5HT existant, dans la mesure où des études pharmacologiques ont révélé d'autres sous-types tels que les récepteurs 5HT4 ainsi que certains récepteurs apparentés au sous-type 5HT1 (récepteurs "5HT1 like"). De plus, des études supplémentaires de biologie moléculaire ont également révélé des hétérogénéités au sein des sous-types 5HT1B/1D.

La présente invention résulte de la mise en évidence de nouveaux polypeptides ayant une activité de récepteur sérotoninergique. Bien qu'appartenant à la famille des récepteurs qui interagissent avec des protéines G, ces nouveaux polypeptides diffèrent des récepteurs sérotoninergiques déjà décrits (5HT1, 5HT2, 5HT3 et 5HT4) du point de vue structural comme du point de vue pharmacologique. Plus particulièrement, l'invention résulte de l'isolement et de la caractérisation de ces nouveaux polypeptides, désignés 5HT5a, ainsi que du matériel génétique permettant leur expression ou leur identification.

Un premier objet de l'invention réside donc dans des polypeptides 5HT5a comprenant la séquence SEQ ID n° 1; l'invention concerne également les peptides 5HT5a comprenant la séquence SEQ ID n° 7 correspondant au récepteur humain.

Préférentiellement, les polypeptides de l'invention sont des polypeptides possédant la capacité de lier la sérotonine. Encore plus préférentiellement, il s'agit de polypeptides ayant une activité de récepteur sérotoninergique. Toujours selon un mode préféré, les polypeptides de l'invention sont susceptibles d'être reconnus par des anticorps reconnaissant la séquence peptidique SEQ ID n° 1 complète.

Comme indiqué dans les exemples, le polypeptide 5HT5a peut être exprimé dans différents types cellulaires pour former un récepteur sérotoninergique fonctionnel.

Les polypeptides de l'invention peuvent être obtenus par expression dans un hôte cellulaire d'une séquence nucléotidique telle que décrite ci-dessous, par synthèse chimique, sur la base de la séquence SEQ ID n° 1 en utilisant les techniques connues de l'homme du métier, ou par une combinaison de ces techniques.

Dans ce qui suit, les polypeptides de l'invention tels que définis ci-dessus sont désignés par polypeptides 5HT5a.

La présente invention a également pour objet toute séquence nucléotidique codant pour un polypeptide 5HT5a. Plus préférentiellement, il s'agit d'une séquence choisie parmi :
(a) la séquence nucléotidique SEQ ID n° 1 ou son brin complémentaire,
(b) toute séquence hybridant avec une séquence (a) et codant pour un polypeptide tel que défini précédemment, et,
(c) les séquences dérivées des séquences (a) et (b) en raison de la dégénérescence du code génétique.

Selon un mode de réalisation particulier, l'invention a pour objet une séquence nucléotidique comprenant la séquence SEQ ID NO 7.

Les différentes séquences nucléotidiques de l'invention peuvent être d'origine artificielle ou non. Il peut s'agir de séquences génomiques, d'ADNc, d'ARN, de séquences hybrides ou de séquences synthétiques ou semi-synthétiques. Ces séquences peuvent être obtenues par exemple par criblage de banques d'ADN (banque d'ADNc, banque d'ADN génomique) au moyen de sondes élaborées sur la base de la séquence SEQ ID n° 1. De telles banques peuvent être préparées à partir de cellules de différentes origines par des techniques classiques de biologie moléculaire connues de l'homme du métier. Les séquences nucléotidiques de l'invention peuvent également être préparées par synthèse chimique, notamment selon la méthode des phosphoramidites, ou encore par des méthodes mixtes incluant la modification chimique ou enzymatiqe de séquences obtenues par criblage de banques.

Les séquences nucléotidiques de l'invention peuvent être utilisées pour la production des polypeptides 5HT5a tels que définis précédemment. Dans ce cas, la partie codant pour ledit polypeptide est généralement placée sous le contrôle de signaux permettant son expression dans un hôte cellulaire. Le choix de ces signaux (promoteurs, terminateurs, etc) peut varier en fonction de l'hôte cellulaire utilisé. A cet effet, les séquences nucléotidiques de l'invention peuvent faire partie d'un vecteur, qui peut être à réplication autonome ou intégratif. Plus particulièrement, des vecteurs à réplication autonome peuvent être préparés en utilisant des séquences à réplication autonome chez l'hôte choisi. S'agissant des vecteurs intégratifs, ceux-ci peuvent être préparés par exemple en utilisant des séquences homologues à certaines régions du génome de l'hôte, permettant, par recombinaison homologue, l'intégration du vecteur. Les hôtes cellulaires utilisables pour la production des polypeptides 5HT5a de l'invention par voie recombinante sont aussi bien des hôtes eucaryotes que procaryotes. Parmi les hôtes eucaryotes qui conviennent, on peut citer les cellules animales, les levures, ou les champignons. En particulier, s'agissant de levures, on peut citer les levures du genre *Saccharomyces, Kluyveromyces, Pichia, Schwanniomyces*, ou *Hansenula*. S'agissant de cellules animales, on peut citer les cellules COS, CHO, C127, NIH-3T3, etc. Parmi les champignons, on peut citer plus particulièrement *Aspergillus* ssp. ou *Trichoderma* ssp. Comme hôtes procaryotes, on préfère utiliser les bactéries suivantes *E. coli, Bacillus*, ou *Streptomyces*.

Les séquences nucléotidiques de la présente invention sont également utilisables dans le domaine pharmaceutique, soit pour la réalisation de séquences antisens utilisables dans le cadre d'une thérapie génique, soit encore pour la réalisation de sondes permettant la détection, par des expériences d'hybridation, de l'expression de récepteurs sérotoninergiques dans des échantillons biologiques et la mise en évidence d'anomalies génétiques (polymorphisme, mutations) ou d'expressions aberrantes.

L'inhibition de l'expression de certains gènes par des oligonucléotides antisens s'est avérée être une stratégie prometteuse dans le contrôle de l'activité d'un gène. Les oligonucléotides antisens sont des oliogonucléotides de petite taille, complémentaire du brin codant d'un gène donné, et de ce fait capables d'hybrider spécifiquement avec l'ARNm transcrit, inhibant sa traduction en protéine. L'invention a ainsi pour objet les oligonucléotides antisens capables d'inhiber au moins partiellement la production de polypeptides 5HT5a tels que définis précédemment. De tels oligonucléotides peuvent être constitués par les séquences nucléotidiques définies ci-avant.

Comme indiqué ci-dessus, l'invention permet également la réalisation de sondes nucléotidiques, synthétiques ou non, capables de s'hydrider avec les séquences nucléotidiques définies ci-avant qui codent pour des polypeptides 5HT5a de l'invention, ou avec les ARNm correspondant. De telles sondes peuvent être utilisées *in vitro* comme outil de diagnostic, pour la détection de l'expression d'un récepteur sérotoninergique 5HT5a, ou encore pour la mise en évidence d'anomalies génétiques (mauvais épissage, polymorphisme, mutations ponctuelles, etc). Compte tenu des activités multiples de la sérotonine, les sondes de l'invention peuvent ainsi permettre d'identifier des affections neurologique, cardiovasculaire ou psychiatrique comme étant liées aux récepteurs 5HT5a. Ces sondes peuvent également être utilisées pour la mise en évidence et l'isolement de séquences d'acides nucléiques homologues codant pour des polypeptides 5HT5a tels que définis précédemment, à partir d'autres sources cellulaires et préférentiellement de cellules d'origines humaines, ainsi qu'illustré dans les exemples. Les sondes de l'invention comportent généralement au moins 10 bases, et elles peuvent comporter jusqu'à l'intégralité de la séquence SEQ ID n° 1 ou SEQ ID n° 7 ou de leur brin complémentaire. Préférentiellement, ces sondes sont, préalablement à leur utilisation, marquées. Pour cela, différentes techniques connues de l'homme du métier peuvent être employées (marquage radioactif, enzymatique, etc). Les conditions d'hybridation dans lesquelles ces sondes peuvent être utilisées sont indiquées dans les techniques générales de clonage ci-après ainsi que dans les exemples.

Un autre objet de l'invention concerne les cellules recombinées capables d'exprimer à leur surface un polypeptide 5HT5a tel que défini ci-avant. Ces cellules peuvent être obtenues par introduction d'une séquence nucléotidique telle que définie ci-dessus codant pour un polypeptide de l'invention, puis culture desdites cellules dans des conditions d'expression de ladite séquence.

Les cellules recombinées selon l'invention peuvent être aussi bien des cellules eucaryotes que procaryotes. Parmi les cellules eucaryotes qui conviennent, on peut citer les cellules animales, les levures, ou les champignons. En particulier, s'agissant de levures, on peut citer les levures du genre *Saccharomyces, Kluyveromyces, Pichia, Schwanniomyces*, ou *Hansenula*. S'agissant de cellules animales, on peut citer les cellules COS, CHO, C127, NIH-3T3, etc. Parmi les champignons, on peut citer plus particulièrement *Aspergillus* ssp. ou *Trichoderma* ssp. Comme cellules procaryotes, on préfère utiliser les bactéries suivantes *E.coli, Bacillus,* ou *Streptomyces.* Les cellules ainsi obtenues peuvent être utilisées pour mesurer la capacité de différentes molécules à se comporter comme ligand ou comme modulateur de l'activité des polypeptides de l'invention. Plus particulièrement, elles peuvent ainsi être utilisées dans un procédé de mise en évidence et d'isolement de ligands ou de modulateur de l'activité des polypeptides de l'invention, et, plus préférentiellement, d'agonistes et d'antagonistes de la sérotonine.

Un autre objet de l'invention concerne donc un procédé de mise en évidence et/ou d'isolement de ligands des polypeptides 5HT5a de l'invention, selon lequel on réalise les étapes suivantes :
- on met en contact une molécule ou un mélange contenant différentes molécules, éventuellement non-identifiées, avec une cellule recombinée telle que décrite ci-dessus exprimant à sa surface un polypeptide de l'invention dans des conditions permettant l'interaction entre ledit polypeptide de l'invention et ladite molécule dans le cas où celle-ci posséderait une affinité pour ledit polypeptide, et,
- on détecte et/ou isole les molécules liées au dit polypeptide de l'invention.

Dans un mode particulier, ce procédé de l'invention est adapté à la mise en évidence et/ou l'isolement d'agonistes et d'antagonistes de la sérotonine pour les polypeptides 5HT5a.

Un autre objet de l'invention concerne un procédé de mise en évidence et/ou d'isolement de modulateurs des polypeptides 5HT5a de l'invention, selon lequel on réalise les étapes suivantes :
- on met en contact une molécule ou un mélange contenant différentes molécules, éventuellement non-identifiées, avec une cellule recombinée telle que décrite ci-dessus exprimant à sa surface un polypeptide de l'invention, en présence de 5HT, dans des conditions permettant l'interaction entre ledit polypeptide de l'invention et le 5HT, et,
- on détecte et/ou isole les molécules capables de moduler l'activité du 5HT sur ledit polypeptide de l'invention.

D'autres avantages de la présente invention apparaîtront à la lecture des exemples qui suivent, qui doivent être considérés comme illustratifs et non limitatifs.

### Légende des figures

SEQ ID n° 1 : Séquences nucléotidique et peptidique du récepteur 5HT5a. L'ADNc de 4 kb a été séquencé sur les 2 brins depuis le site EcoRI jusqu'à la position 1685. Les 2300 nucléotides restant n'ont pas été séquencés à l'exception de l'extrémité 3' contenant la queue polyA.
Figure 2 : Pourcentages d'homologie de séquence peptidique entre le récepteur 5HT5a SEQ ID n° 1 et d'autres récepteurs de la famille des récepteurs couplés à des protéines G. Les homologies ont été calculées sur les séquences conservées : le domaine transmembranaire et ses boucles de connection.
Figure 3 : Courbe de saturation du [¹²⁵I]-LSD aux membranes des cellules Cos-7 exprimant le récepteur 5HT5a. Les membranes ont été incubées avec des concentrations de ligand allant de 50 pM à 1,25 nM, avec ou sans 10 µM de 5HT. La liaison spécifique est représentée. L'encart représente l'analyse en Scatchard des résultats.
Figure 4 : Mise en évidence de séquences homologues : (a) par Northern blot sur des ARNm polyA (5 µg) de différents tissus; (b) par PCR sur des ARN totaux (1 µg) de différents tissus.
   Table 1 : Profil pharmacologique du récepteur 5HT5a. Les résultats correspondent à des expériences de compétition pour la liaison du [¹²⁵I]-LSD soit aux membranes des cellules Cos-7 exprimant le récepteur 5HT5a de manière transitoire, soit aux membranes des cellules NS4 exprimant le récepteur 5HT5a de manière stable. Les valeurs d'IC50 (correspondant à la concentration en ligand nécessaire pour déplacer 50 % du [¹²⁵I]-LSD lié) ont été calculées expérimentalement et converties en Ki selon l'équation suivante : Ki = IC50/(1 + C/Kd) dans laquelle C est la concentration en [¹²⁵I]-LSD et Kd est la constante de dissociation du [¹²⁵I]-LSD (308 pM). Les nombres entre parenthèses correspondent au nombre d'expériences indépendantes réalisées, chaque point étant réalisé en triple.

### Techniques générales de clonage

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extractions de protéines au phénol ou au phénol-chloroforme, la précipitation d'ADN en milieu salin par de l'éthanol ou de l'isopropanol, la transformation dans *Escherichia coli*, etc, sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les enzymes de restriction ont été fournies par New England Biolabs (Biolabs), Bethesda Research Laboratories (BRL) ou Amersham et sont utilisées selon les recommandations des fournisseurs.

Pour les ligatures, les fragments d'ADN sont séparés selon leur taille par électrophorèse en gels d'agarose ou d'acrylamide, extraits au phénol ou par un mélange phénol/chloroforme, précipités à l'éthanol puis incubés en présence de l'ADN ligase du pliage T4 (Biolabs) selon les recommandations du fournisseur.

Le remplissage des extrémités 5' proéminentes est effectué par le fragment de Klenow de l'ADN Polymérase I d'*E. coli* (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée *in vitro* par oligodéoxynucléotides synthétiques est effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354 ; Mullis K.B. et Faloona F.A., Meth. Enzym. 155 (1987) 335-350] est effectuée en utilisant un "DNA thermal cycler" (Perkin Elmer Cetus) selon les spécifications du fabricant.

La vérification des séquences nucléotidiques est effectuée par la méthode développée par Sanger et al. [Proc. Natl. Acad. Sci. USA, 74 (1977) 5463-5467] en utilisant le kit distribué par Amersham.

Pour les expériences d'hybridation, les conditions de stringence normales sont généralement les suivantes : hybridation : 3 x SCC en présence de 5 x Denhart's à 65°C ; lavage : 0,5 x SSC à 65°C.

### 1. Isolement du récepteur 5HT5a

Les comparaisons de séquences entre les différents récepteurs sérotoninergiques connus font apparaître une certaine conservation, particulièrement dans certaines régions transmembranaires potentielles telles que les domaines III et IV. Dans le but de mettre en évidence et d'isoler un nouveau récepteur, trois oligonucléotides dégénérés correspondant à ces deux régions ont été préparés, puis utilisés dans une série de réactions de PCR sur une préparation d'ARN de cerveau de rat. La séquence des oligonucléotides dégénérés (i)-(iii) est donnée dans les séquences SEQ ID n° 2-4.

Les réactions de PCR ont été réalisées de la manière suivante : 5 µg d'ARN de cerveau de rat adulte ont été soumis à une réaction de transcription inverse en présence de 500 ng d'oligonucléotide (i) et de 200 unités de transcriptase inverse MMLV (BRL). La moitié du produit de cette réaction a ensuite été soumise à 30 cycles d'amplification en présence de 5 unités de polymérase Taq (Cetus) et de 1 µg d'oligonucléotide (i) et d'oligonucléotide (ii). 1/20e du produit de cette réaction a ensuite été soumis à 30 cycles d'amplification supplémentaires en présence des oligonucléotides (i) et (iii). Les produits ainsi obtenus ont été digérés avec les enzymes BamHI et HindIII, insérés aux sites correspondant du plasmide Bluescript (Stratagène), et Séquencés. L'un des fragments ainsi obtenus, présentant une certaine homologie avec les récepteurs sérotoninergiques, a été marqué par "random priming" (Feinberg et Vogelstein, Analytical Biochemistry 132 (1984) 6) et utilisé comme sonde pour cribler une banque de cDNA de cerveau de rat construite dans le phage UniZap (Stratagème). Parmi les phages positifs obtenus, l'un d'entre-eux, dénommé λNS et porté par le plasmide pNS, contenait un insert de 4 kb. Ce phage a été isolé, et son insert a ensuite été introduit dans le plasmide Bluescript. La séquence de ce fragment a été déterminée sur 1,6 kb environ sur les 2 brins en utilisant la technique des dideoxynucléotides au moyen d'oligonucléotides synthétiques.

La séquence ainsi obtenue est présentée sur la séquence SEQ ID n° 1. Elle montre que l'ADNc isolé porte une phase de lecture ouverte de 357 acides aminés. Par ailleurs, l'analyse d'hydrophobicité montre que cette protéine porte sept domaines hydrophobes, une particularité rencontrée chez les membres de la famille des récepteurs couplés à des protéines G. L'extrémité N-terminale contient par ailleurs 2 sites de N-glycosylation, et le domaine cytoplasmique présumé contient les sites consensus de phosphorylation par les protéines kinases C et A.

### 2. Etude d'homologies de séquence

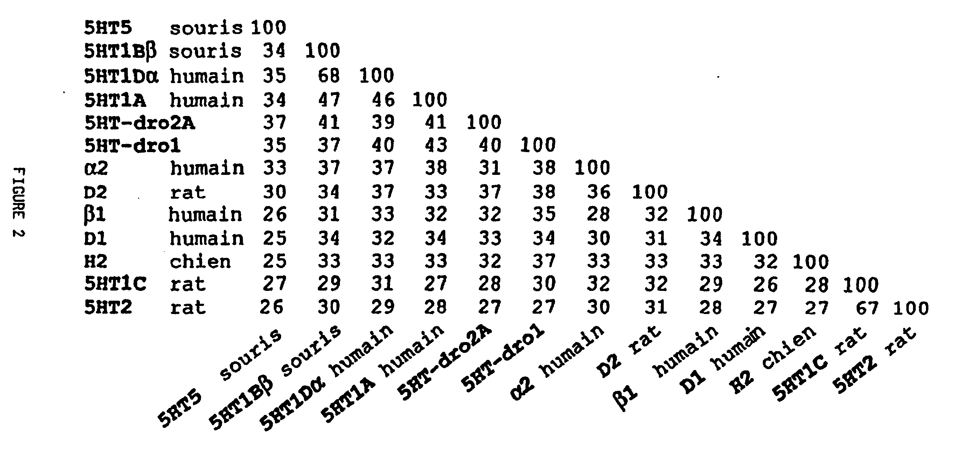

La séquence du récepteur 5HT5a isolé ci-dessus a été comparée avec les séquences des récepteurs couplés à des protéines G suivants : 5HT1B, 5HT1D, 5HT1A, 5HT-dro2A, 5HT-dro1, α2, D2, β1, D1, H2, 5HT1C et 5HT2. Ces expériences ont révélé une certaine homologie dans le domaine transmembranaire potentiel et dans certaines boucles, mais pas dans les régions terminales ni dans la troisième boucle cytoplasmique. La figure 2 donne les % d'homologie au niveau des régions conservées.

Comme il ressort de cette figure, l'homolgie avec les récepteurs connus est faible, le meilleur résultat étant obtenu avec le récepteur sérotoninergique de drosophile HT-dro2A (37 % d'homologie).

### 3. Expression du récepteur 5HT5a dans les cellules Cos-7 et caractérisation pharmacologique

Le fragment d'ADNc isolé dans l'exemple 1 a été inséré dans un vecteur d'expression eucaryote, qui a été utilisé pour transfecter des cellules Cos-7. Les membranes des cellules transfectées obtenues ont ensuite été préparées et testées pour leur capacité à lier certains ligands sérotoninergiques marqués.

L'ADNc de 4 kb codant pour le récepteur 5HT5a a été isolé à partir du plasmide pNS sous forme d'un fragment EcoRI-XhoI, puis inséré aux sites correspondants du vecteur p513. Le vecteur p513 dérive du vecteur pSG5 [Green et al., NucL Acids Res. 16 (1988) 369] par addition d'un multisite de clonage. Le vecteur recombinant ainsi obtenu désigné p513NS a ensuite été utilisé (20 µg par plaque de 10 cm) pour transfecter les cellules Cos-7 en présence de phosphate de calcium.

48 heures après la transfection, les cellules recombinantes sont récoltées et les membranes sont préparées selon la technique décrite par Amlaiky et Caron [J. Biol. Chem. 260 (1985) 1983]. Des expériences de liaison à saturation et de compétition ont ensuite été réalisées sur ces membranes en présence des ligands radiomarqués suivants : [¹²⁵I]-LSD ; [¹²⁵I]-cyanopindolol ; [³H]-8-OH-DPAT et [³H]-spiperone. Pour cela, les échantillons de membrane (10-20 µg de protéines) ont été incubés 10 minutes à 37°C en présence du ligand dans un volume final de 250 µl de tampon Tris-HCl 50 mM (pH 7,4). La réaction est ensuite stppée par filtration sous vide sur filtres en fibre de verre Whatman GF/C, et rinçage 4 fois avec 4 ml de tampon Tris-HCl 50 mM (pH 7,4). La liaison non-spécifique a été déterminée en présence de 10 µM de 5HT. La radioactivité a été mesurée avec un compteur γ.

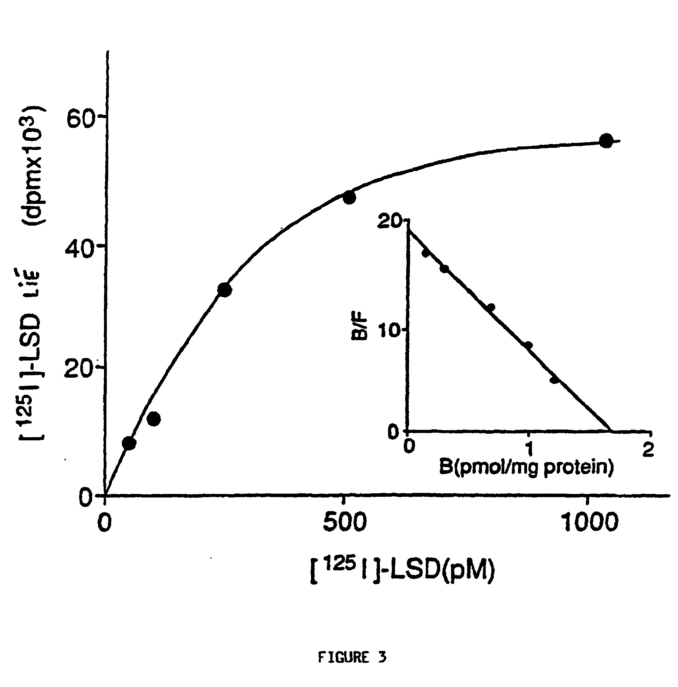

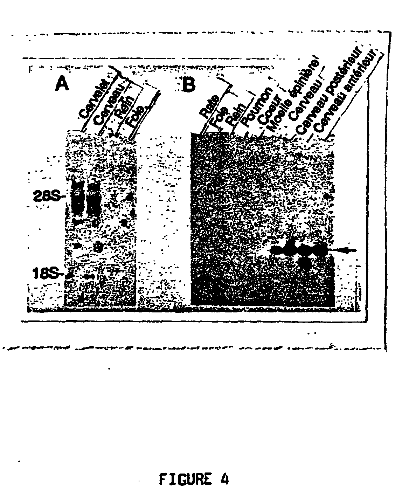

Les résultats obtenus montrent que, bien que le [¹²⁵I]-cyanopindolol ; le [³H]-8-OH-DPAT et le [³H]-spiperone ne lient pas les membranes préparées, le [¹²⁵I]-LSD présente un site de liaison saturable avec un Kd = 340 pM et un Bmax = 1,6 pmol/mg de protéines membranaires (figure 3). Dans une exprérience contrôle, il a par ailleurs été montré que le [¹²⁵I]-LSD ne liait pas les cellules Cos-7 transfectées par le plasmide p513.

Pour déterminer le profil pharmacologique de ce récepteur, le [¹²⁵I]-LSD lié aux membranes a été déplacé en présence de différentes drogues sérotoninergiques (table 1). Ces différentes drogues montrent l'ordre d'efficacité de déplacement suivant : 2-bromo-LSD > ergotamine > 5-CT > methysergide > 5HT = RU24969 > bufotenine > yohimbine = 8-OH-DPAT (table 1). La kétansérine, le (±) pindolol, le sumatriptan, la dopamine et la norépinéphrine sont inactifs.

### 4. Expression du récepteur 5HT5a dans les cellules NIH-3T3 et étude pharmacologique

L'ADNc cloné dans l'exemple 1 a également été exprimé dans les cellules NIH-3T3, qui n'expriment aucun récepteur sérotoninergique de manière endogène. Pour cela, le vecteur d'expression recombinant décrit en 3. ci-dessus a été utilisé. Il a été introduit (20 µg par plaque de 10 cm) dans les cellules NIH-3T3 par transfection en présence de phosphate de calcium, en même temps que le vecteur pRSVnéo [Gorman et al., Science 221 (1983) 551], portant le gène de résistance au G418 (1 µg par plaque de 10 cm). Les clones transformants ont été sélectionnés en présence de 0,5 mg de G418. Les clones isolés ont ensuite été amplifiés et les RNA totaux de ces clones ont été préparés et analysés en Northern Blot pour l'expression d'ARNm du 5HT5a. 2 clones ont ainsi été sélectionnés, NS1 et NS4, exprimant respectivement des niveaux élevés et faibles d'ARNm du 5HT5a.

Les membranes des cellules de ces clones ont ensuite été préparées et testées dans les conditions décrites ci-dessus pour leur capacité à lier certains ligands sérotoninergiques marqués, et pour déterminer l'affinité des récepteurs pour lesdits ligands.

Les résultats obtenus montrent que les membranes de ces 2 clones possèdent des sites de liaison de haute affinité pour le [¹²⁵I]-LSD, indiquant que ces 2 clones expriment des récepteurs 5HT5a. Une expérience contrôle a en effet montré que les cellules NIH-3T3 non transfectées étaient incapables de lier le [¹²⁵I]-LSD. Différentes expériences de déplacement ont ensuite été réalisées (table 1). Dans le cas du 5HT, du 5CT, du sumatriptan et du 8-OH-DPAT, les courbes de compétition obtenues étaient biphasiques, et une analyse des résultats a révélé 2 composants de liaison, l'un avec une affinité élevée et l'autre avec une affinité plus faible.

### 5. Recherche de séquences homologues dans d'autres tissus

La séquence nucléotidique SEQ ID n° 1 a ensuite été utilisée pour la mise en évidence de séquences homologues à partir d'autres tissus. Pour cela, trois techniques ont été utilisées :
- l'hybridation en Northern blot,
- la PCR
- l'hybridation *in situ*.

Les tissus utilisés pour la recherche de séquences homologues sont les suivants d'origine marine : cerveau, cervelet, rein, foie, moelle épinière, rate, poumon et coeur.

### 5.1. Recherche par hybridation en Northern blot.

Les ARNm-polyA ont été préparés à partir des tissus indiqués ci-dessus selon la technique décrite par Cathala et al. (DNA 2(4) (1983)), suivie d'un passage sur colonne d'oligodT-cellulose. Ces ARNm ont ensuite été fractionnés sur gel d'agarose-formaldéhyde à 1%, puis transférés sur filtre de nitro-cellulose. La sonde utilisée pour l'hybridation correspond au fragment EcoRI-XhoI de 4 kb entier décrit dans l'exemple 1 (SEQ ID n° 1), préalablement marqué au ³²P par "random priming". L'hybridation a été réalisée dans des conditions de stringence élevées : 42°C, dans un tampon phosphate de sodium 20 mM (pH 6,5) contenant 50% de formamide, 5 x SSC, 1 x Denhardt's, 0,1 % de SDS et 100 µg/ml d'ARNt. Les lavages ont été effectués à 60°C dans un tampon 0,1 x SSC, 0,1 % SDS.

Cette étude a permis de mettre en évidence trois transcrits homologues dans le cerveau et le cervelet, de 5,8 kb, 5 kb et 4,5 kb (figure 4a).

### 5.2. Recherche par PCR

Pour la recherche par PCR, les sondes (iv) SEQ ID n° 5 et (v) SEQ ID n° 6 ont été utilisées.

La sonde (iv) correspond à la position 1351 sur la séquence SEQ ID n° 1 et la sonde (v) à la position 947.

Les ARN totaux ont été pméparés à partir des différents tissus étudiés, en utilisant la technique décrite par Cathala et al. précitée. 1 µg de ces ARN a été soumis à une transaiption inverse en présence de 200 unités de transcriptase inverse MMLV et de 300 ng de la sonde (iv), pendant 1 heure à 37°C. La moitié du produit de cette réaction a ensuite été amplifiée (30 cycles) en présence de 5 unités de la polymérase Taq (Cetus) et de 500 ng des sondes (iv) et (v). Un aliquot de cette réaction a également été prélevé après 20 cycles d'amplification. Les produits ainsi obtenus ont ensuite été transférés sur filtres de nitro-cellulose et hybridés dans les conditions décrites en 5.1. ci-dessus.

Cette étude a permis de mettre en évidence des fragments d'ADN spécifiques homologues dans la moelle épinière et le cerveau (figure 4b).

### 5.3. Recherche par hybridation in situ

Les expériences d'hybridation *in situ* ont été réalisées sur des sections cryostatées de cerveau de rat adulte (8 semaines environ) selon la technique décrite par Hafen et al. [EMBO J. 2 (1983) 617]. La sonde utilisée pour ces expériences est un ARN simple brin obtenu par transcription en présence de polymérase T7, de [³⁵S]-CTP en utilisant le plasmide pNS comme matrice.

Cette étude a permis de mettre en évidence des séquences homologues selon l'invention dans le cortex cérébral, l'hippocampe et la couche granulaire du cervelet et dans le bulbe olfactif.

### 6. Isolement du récepteur humain

Selon la méthodologie décrite en 5. ci-dessus, le récepteur 5HT5a humain a été cloné.

Pour cela, une banque d'ADN génomique humain a été préparée à partir de placenta, par digestion partielle par l'enzyme Mbol, séparation sur gradients de sels, et sous clonage dans le vecteur Lamda GEM 12 linéarisé par BamHI (bacterie hôte:TAP 90).

La banque ainsi obtenue a ensuite été criblée au moyen de la sonde décrite dans l'exemple 5.1. Les fragments de DNA qui hybrident avec cette sonde ont été isolés, sous clonés dans un plasmide Bluescript, amplifiés, puis séquencés dans les deux sens selon la technique dideoxynucleotide. L'amplification a été réalisée par la technique PCR : 20 cycles en présence de Thermus aquaticus polymerase(2,5 unités; Cetus) et d'oligonudéotides 1 (SEQ ID n° 8), 2 (SEQ ID n° 9) pour la partie A du recepteur en amont de l'intron, d'oligonucléotides 3 (SEQ ID n° 10), 4 (SEQ ID n° 11) pour la partie B du recepteur en aval de l'intron. Le fragment A a été digeré par les enzymes NotI et XhoI et le fragment B par les enzymes EcoRI et XhoI. Ces fragments ont été sous clonés dans un vecteur d'expression P514.

La séquence obtenue est présentée sur la séquence SEQ ID n° 7.

Il est entendu que les même expériences peuvent être répétées en utilisant d'autres tissus et notamment des tissus d'origine humaine, et d'autres sondes. Par ailleurs, les séquences homologues mises en évidence lors de ces expériences peuvent évidemment être ensuite isolées et/ou amplifiées par les techniques classiques de biologie moléculaire.

### LISTE DE SEQUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L' INVENTION: NOUVeaux polypeptides ayant une activite de recepteur serotoninergique, acides nucleiques codant pour ces polypeptides et utilisation.
   (iii) NOMBRE DE SÉQUENCES: 11
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 1686 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Souris
   (ix) CARACTERISTIQUE ADDITIONELLE:
      (A) NOM/CLE: CDS
      (B) EMPLACEMENT: 509..1582
      (D) AUTRES RENSEIGNEMENTS: /product= "Gene recepteur 5HT5A souris"
   (xi) DESCRIPTION DE LA SEQUENCE: SEQ ID NO: 1:
   GGCACGAGGC CGTCTCCAGA AAGCAGGTAT CTACGTGGCT TCCAGTCCCC AACCCCCACC     60
   CCTCGGAGCC ACTGCCGGGA GAGGGGGGAG GTGGGCAAGG AGCAACCCTG GACCAGCGAC    120
   TGTTCTGACG CACTAGCTGA GTTCTGGGCA TCCACCCTGC ACTGGGCGGG GGCGACCCAA    180
   GGATGCTCTG CTGCAGGCGA CCAGACAACA GTCTCCGCCT AGGTGAGGAA CAGCAAGGCA    240
   TGTGATAGCA AAAGGCGGGC CCTGGCTTCT AGATTCAGCC CCTTGAGTCC GCTTTCCATA    300
   TCTCTAAGGA TACCTGGGCT GTGCTGCTTG TAGCCCAGCA CCCTCCTCTC TGCTACAATT    360
   TCCTCCGGAC TCTGACTGGG TGGAGACTGA GGCCAGGTTC TTGGCTCTTA GCAAAATCCT      420
   CTCCATTGGC CATCGGTCGC AAACATCTAG ATTGACTTCA GTGGGCTCGG TGGCAACACA      480
   GTCTAAACAC AGGTGTCCTG GGACAGCA ATG GAT CTG CCT GTA AAC TTG ACC         532
                                  Met Asp Leu Pro Val Asn Leu Thr
                                   1               5
   TCC TTT TCT CTC TCT ACT CCC TCC TCT TTG GAA CCT AAC CGC AGC TTG        580
   Ser Phe Ser Leu Ser Thr Pro Ser Ser Leu Glu Pro Asn Arg Ser Leu
       10                  15                  20
   GAC ACG GAA GTC CTG CGC CCT AGT CGG CCT TTT CTC TCA GCT TTC CGA        628
   Asp Thr Glu Val Leu Arg Pro Ser Arg Pro Phe Leu Ser Ala Phe Arg
   25                  30                  35                  40
   GTG CTA GTC CTG ACT TTG TTG GGC TTT CTA GCT GCG GCC ACA TTC ACT        676
   Val Leu Val Leu Thr Leu Leu Gly Phe Leu Ala Ala Ala Thr Phe Thr
                   45                  50                  55
   TGG AAC CTG CTG GTG CTG GCT ACC ATC CTC AAG GTA CGC ACC TTC CAC        724
   Trp Asn Leu Leu Val Leu Ala Thr Ile Leu Lys Val Arg Thr Phe His
               60                  65                  70
   CGA GTA CCA CAC AAC CTG GTA GCT TCC ATG GCC ATC TCG GAT GTG CTA        772
   Arg Val Pro His Asn Leu Val Ala Ser Met Ala Ile Ser Asp Val Leu
           75                  80                  85
   GTG GCT GTG CTG GTT ATG CCA CTG AGC CTG GTA CAT GAG CTG TCT GGG        820
   Val Ala Val Leu Val Met Pro Leu Ser Leu Val His Glu Leu Ser Gly
       90                  95                  100
   CGC CGC TGG CAG CTG GGC CGG CGT CTA TGC CAG CTG TGG ATC GCA TGT        868
   Arg Arg Trp Gln Leu Gly Arg Arg Leu Cys Gln Leu Trp Ile Ala Cys
   105                 110                 115                 120
   GAC GTG CTC TGC TGT ACT GCC AGC ATC TGG AAT GTG ACA GCA ATA GCA        916
   Asp Val Leu Cys Cys Thr Ala Ser Ile Trp Asn Val Thr Ala Ile Ala
                   125                 130                 135
   CTG GAC CGC TAC TGG TCA ATC ACG CGC CAC CTG GAG TAC ACA CTC CGT        964
   Leu Asp Arg Tyr Trp Ser Ile Thr Arg His Leu Glu Tyr Thr Leu Arg
               140                 145                 150
   ACC CGC AAG CGT GTC TCC AAT GTG ATG ATC CTG CTC ACC TGG GCA CTC       1012
   Thr Arg Lys Arg Val Ser Asn Val Met Ile Leu Leu Thr Trp Ala Leu
           155                 160                 165
   TCC ACT GTC ATC TCT CTG GCT CCA CTG CTA TTT GGC TGG GGA GAG ACT       1060
   Ser Thr Val Ile Ser Leu Ala Pro Leu Leu Phe Gly Trp Gly Glu Thr
       170                 175                 180
   TAT TCT GAG CCC AGT GAG GAA TGC CAA GTC AGT CGC GAG CCT TCC TAC       1108
   Tyr Ser Glu Pro Ser Glu Glu Cys Gln Val Ser Arg Glu Pro Ser Tyr
   185                 190                 195                 200
   ACC GTG TTC TCC ACC GTG GGT GCC TTC TAC CTG CCG CTG TGG CTG GTG       1156
   Thr Val Phe Ser Thr Val Gly Ala Phe Tyr Leu Pro Leu Trp Leu Val
                   205                 210                 215
   CTC TTT GTG TAC TGG AAA ATT TAC AGG GCG GCG AAA TTC CGC ATG GGC       1204
   Leu Phe Val Tyr Trp Lys Ile Tyr Arg Ala Ala Lys Phe Arg Met Gly
               220                 225                 230
   TCC AGG AAG ACT AAC AGC GTC TCC CCC GTA CCC GAA GCT GTG GAG GTG      1252
   Ser Arg Lys Thr Asn Ser Val Ser Pro Val Pro Glu Ala Val Glu Val
           235                 240                 245
   AAG AAT GCT ACA CAA CAT CCC CAG ATG GTG TTC ACG GCC CGC CAT GCC      1300
   Lys Asn Ala Thr Gln His Pro Gln Met Val Phe Thr Ala Arg His Ala
       250                 255                 260
   ACC GTC ACC TTC CAG ACA GAA GGG GAT ACG TGG AGG GAG CAG AAG GAG      1348
   Thr Val Thr Phe Gln Thr Glu Gly Asp Thr Trp Arg Glu Gln Lys Glu
   265                 270                 275                 280
   CAA AGG GCA GCC CTC ATG GTG GGC ATC CTC ATC GGA GTG TTT GTG CTC      1396
   Gln Arg Ala Ala Leu Met Val Gly Ile Leu Ile Gly Val Phe Val Leu
                   285                 290                 295
   TGC TGG TTC CCT TTC TTC GTC ACA GAG CTC ATC AGT CCC CTG TGT TCC      1444
   Cys Trp Phe Pro Phe Phe Val Thr Glu Leu Ile Ser Pro Leu Cys Ser
               300                 305                 310
   TGG GAC GTC CCT GCC ATC TGG AAG AGC ATC TTC CTG TGG TTG GGC TAT      1492
   Trp Asp Val Pro Ala Ile Trp Lys Ser Ile Phe Leu Trp Leu Gly Tyr
           315                 320                 325
   TCT AAT TCC TTC TTC AAC CCA CTC ATC TAC ACA GCA TTC AAC AGG AGC      1540
   Ser Asn Ser Phe Phe Asn Pro Leu Ile Tyr Thr Ala Phe Asn Arg Ser
       330                 335                 340
   TAC AGC AGT GCT TTC AAG GTC TTC TTC TCC AAG CAA CAA TGAGAGACCA       1589
   Tyr Ser Ser Ala Phe Lys Val Phe Phe Ser Lys Gln Gln
   345                 350                 355
   CATGGGAGTG CCTTCTTCCC ATAGCTTGTA GCTCAGTGGG TTATATTGTC CCATGAACCT    1649
   TTGCAGGCTG CCCAGCTGTC TTTGAGGACA AGATCCA                             1686
(2) INFORMATION POUR LA SEQ ID NO: 2:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION: linéaire
   (ii) TYPE DE MOLECULE: ADNc
   (vi) ORIGINE:
      (A) ORGANISME: oligonucleotide (i)
   (xi) DESCRIPTION DE LA SEQUENCE: SEQ ID NO: 2:
   AGAACTAGTG GATCCAARAA NGGNARCCAR CA                                  32
(2) INFORMATION POUR LA SEQ ID NO: 3:
   (i) CARACTERISTIQUES DE LA SÉQUENCE:
      (A) LONGUEUR: 32 paires de bases
      (B) TYPE: acide nucléique
      (C) NOMBRE DE BRINS: simple
      (D) CONFIGURATION: linéaire
   (ii) TYPE DE MOLECULE: ADNc
   (vi) ORIGINE:
      (A) ORGANISME: oligonucleotide (ii)
   (xi) DESCRIPTION DE LA SEQUENCE: SEQ ID NO: 3:
   CTTGATATCG AATTCGAYRT NCTNTGYTGY AC                                    32
(2) INFORMATION POUR LA SEQ ID NO: 4:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION: linéaire
   (ii) TYPE DE MOLECULE: ADNc
   (vi) ORIGINE:
      (A) ORGANISME: oligonucleotide (iii)
   (xi) DESCRIPTION DE LA SEQUENCE: SEQ ID NO: 4:
   GGTATCGATA AGCTTATHGC YCTNGAYMGN TA                                    32
(2) INFORMATION POUR LA SEQ ID NO:5:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADNc
   (vi) ORIGINE:
      (A) ORGANISME: oligonucleotide (iv)
   (xi) DESCRIPTION DE LA SEQUENCE: SEQ ID NO: 5:
   CTTCTGCTCC CTCCACGTAT C                                                21
(2) INFORMATION POUR LA SEQ ID NO: 6:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADNc
   (vi) ORIGINE:
      (A) ORGANISME: oligonucleotide (v)
   (xi) DESCRIPTION DE LA SEQUENCE: SEQ ID NO: 6:
   CGCCACCTGG AGTACACACT C 21
(2) INFORMATION POUR LA SEQ ID NO: 7:
   (i) CARACTERISTIQUES DE LA SEQUENCE:
      (A) LONGUEUR: 1074 paires de bases
      (B) TYPE: acide nucléique
      (C) NOMBRE DE BRINS: double
      (D) CONFIGURATION: linéaire
   (il) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 1..1074
      (D) AUTRES RENSEIGNEMENTS: /product= "GENE DU RECEPTEUR 5HT5A HUMAIN"
   (xi) DESCRIPTION DE LA SEQUENCE: SEQ ID NO: 7:
(2) INFORMATION POUR LA SEQ ID NO:8:
   (i) CARACTERISTIQUES DE LA SEQUENCE:
      (A) LONGUEUR: 43 paires de bases
      (B) TYPE: acide nucléique
      (C) NOMBRE DE BRINS: simple
      (D) CONFIGURATION: linéaire
   (ii) TYPE DE MOLECULE: ADNc
   (vi) ORIGINE:
      (A) ORGANISME: oligonucleotide 1
   (xi) DESCRIPTION DE LA SEQUENCE: SEQ ID NO: 8:
   GCATGCGCGC GGCCGCGGCA CCATGGATTT ACCTGTGAAC CTA                   43
(2) INFORMATION POUR LA SEQ ID NO: 9:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADNc
   (vi) ORIGINE:
      (A) ORGANISME: oligonudeotide 2
   (xi) DESCRIPTION DE LA SEQUENCE: SEQ ID NO: 9:
   TTCGGATATC GGTGAGACGC                                             20
(2) INFORMATION POUR LA SEQ ID NO: 10:
   (i) CARACTERISTIQUES DE LA SEQUENCE:
      (A) LONGUEUR: 41 paires de bases
      (B) TYPE: acide nucléique
      (C) NOMBRE DE BRINS: simple
      (D) CONFIGURATION: linéaire
   (ii) TYPE DE MOLECULE: ADNc
   (vi) ORIGINE:
      (A) ORGANISME: oligonucleotide 3
   (xi) DESCRIPTION DE LA SEQUENCE: SEQ ID NO: 10:
   CCGATATCCG AAGCTGTGGA GGTGAAGGAC TCTGCCCAAC A 41
(2) INFORMATION POUR LA SEQ ID NO: 11:
   (i) CARACTERISTIQUES DE LA SEQUENCE:
      (A) LONGUEUR: 37 paires de bases
      (B) TYPE: acide nucléique
      (C) NOMBRE DE BRINS: simple
      (D) CONFIGURATION: linéaire
   (ii) TYPE DE MOLECULE: ADNc
   (vi) ORIGINE:
      (A) ORGANISME: oligonucleotide 4
   (xi) DESCRIPTION DE LA SEQUENCE: SEQ ID NO: 11:
   AACCCGGGCT TAAGTCAGTG TTGCCTAGAA AAGAAGT 37

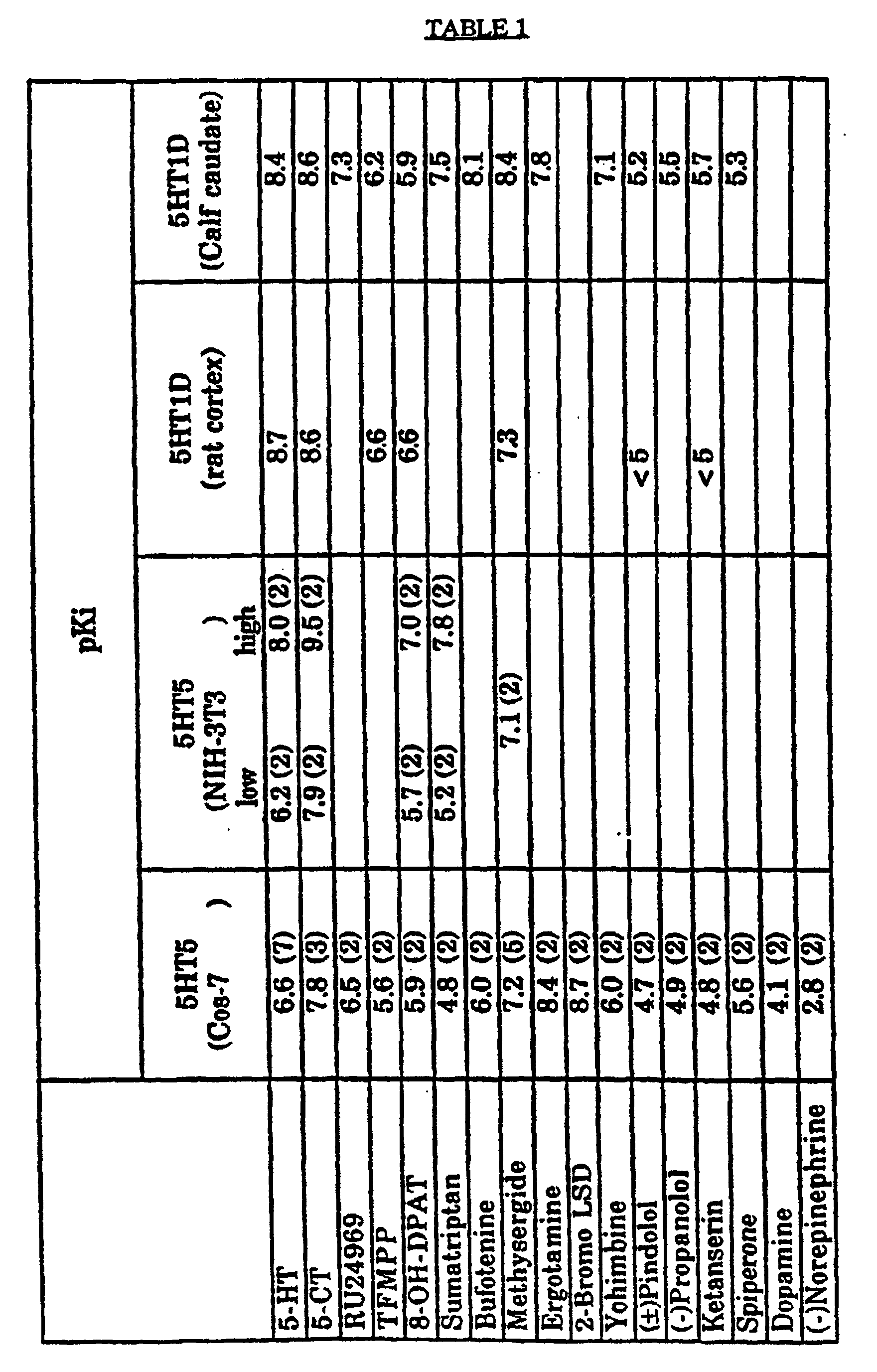

## Revendications

1. Polypeptide comprenant la séquence peptidique SEQ ID N° 1.

2. Polypeptide selon la revendication 1 **caractérisé en ce qu'**il possède la capacité de lier la sérotonine.

3. Polypeptide selon la revendication 2 **caractérisé en ce qu'**il possède une activité de récepteur sérotoninergique.

4. Polypeptide selon l'une des revendications 1 à 3 **caractérisé en ce qu'**il peut être reconnu par des anticorps reconnaissant la séquence peptidique SEQ ID N° 1.

5. Polypeptide comprenant la séquence peptidique SEQ ID N° 7.

6. Séquence nucléotidique codant pour un polypeptide selon l'une des revendications 1 à 5.

7. Séquence selon la revendication 6, **caractérisée en ce qu'**elle est choisie parmi :
(a) la séquence nucléotidique SEQ ID N° 1 ou son brin complémentaire,
(b) toute séquence hybridant avec une séquence (a) et codant pour un polypeptide selon l'une des revendications 1 à 5, et,
(c) les séquences dérivées des séquences (a) et (b) en raison de la dégénérescence du code génétique.

8. Séquence selon la revendication 7 **caractérisée en ce qu'**elle comprend la séquence nucléotidique SEQ ID N° 7.

9. Séquence selon la revendication 7 **caractérisée en ce qu'**elle est choisie parmi les séquences génomiques, d'ADNc, d'ARN, les séquences hybrides ou les séquences synthétique ou semi-synthétiques.

10. Séquence selon l'une des revendications 6 à 9, **caractérisée en ce que** la partie codant pour ledit polypeptide est placée sous le contrôle de signaux permettant son expression dans un hôte cellulaire.

11. Oligonucléotide antisens capable d'inhiber au moins partiellement la production de polypeptide selon l'une des revendications 1 à 5, **caractérisé en ce qu'**il est constitué par une séquence nucléotidique selon la revendication 7.

12. Sonde nucléotidique capable de s'hybrider avec une séquence selon la revendication 7 ou avec l'ARNm correspondant, **caractérisée en ce qu'**elle comporte la séquence nucléotidique SEQ ID N° 1, la séquence nucléotidique SEQ ID N° 7 ou leurs brins complémentaires.

13. Sonde nucléotidique capable de s'hybrider avec une séquence selon la revendication 7 ou avec l'ARNm correspondant, **caractérisée en ce qu'**elle est choisie parmi les séquences SEQ ID N° 5, 6, 8-11.

14. Utilisation in vitro d'une sonde selon la revendication 12 ou 13 comme agent pour la détection de l'expression d'un récepteur sérotoninergique 5HT5a ; ou pour la mise en évidence d'anomalies génériques (mauvais épissage, polymorphisme, mutations ponctuelles, etc) ; ou pour identifier des affections neurologique, cardiovasculaire ou psychiatrique comme étant liées aux récepteurs 5HT5a ; ou encore pour la mise en évidence et l'isolement de séquences d'acides nucléiques homologues codant pour des polypeptides 5HT5a.

15. Vecteur d'expression, **caractérisé en ce qu'**il contient une séquence nucléotidique selon l'une des revendications 6 à 10.

16. Cellule recombinée capable d'exprimer à sa surface un polypeptide selon l'une des revendications 1 à 5, **caractérisée en ce qu'**elle contient un vecteur d'expression selon la revendication 15.

17. Cellule selon la revendication 16, **caractérisée en ce qu'**elle est choisie parmi les cellules eucaryotes ou procaryotes.

18. Procédé de mise en évidence et/ou d'isolement de ligands des polypeptides tels que définis dans les revendications 1 à 5, **caractérisé en ce que** l'on réalise les étapes suivantes :
- on met en contact une molécule ou un mélange contenant différentes molécules, éventuellement non-identifiées, avec une cellule recombinée selon la revendication 16 exprimant à sa surface un polypeptide tel que défini dans les revendications 1 à 5 dans des conditions permettant l'interaction entre ledit polypeptide et ladite molécule dans le cas où celle-ci posséderait une affinité pour ledit polypeptide, et,
- on détecte et/ou isole les molécules liées audit polypeptide.

19. Procédé selon la revendication 18 pour la mise en évidence et/ou l'isolement d'agonistes ou d'antagonistes de la sérotonine.

20. Procédé de mise en évidence et/ou d'isolement de modulateurs des polypeptides tels que définis dans les revendications 1 à 5, **caractérisé en ce que** l'on réalise les étapes suivantes :
- on met en contact une molécule ou un mélange contenant différentes molécules, éventuellement non-identifiées, avec une cellule recombinée selon la revendication 16 exprimant à sa surface un polypeptide tel que défini dans les revendications 1 à 5, en présence de 5HT, dans des conditions permettant l'interaction entre ledit polypeptide et le 5HT, et,
- on détecte et/ou isole les molécules capables de moduler l'activité du 5HT sur ledit polypeptide.

## Patentansprüche

1. Polypeptid, umfassend die Peptidsequenz SEQ ID Nr. 1.

2. Polypeptid nach Anspruch 1, **dadurch gekennzeichnet, dass** es die Fähigkeit besitzt, Serotonin zu binden.

3. Polypeptid nach Anspruch 2, **dadurch gekennzeichnet, dass** es eine Serotoninrezeptoraktivität aufweist.

4. Polypeptid nach einem der Ansprüche 1 bis 3, **dadurch gekennzeichnet, dass** es von Antikörpern, die die Peptidsequenz SEQ ID Nr. 1 erkennen, erkannt werden kann.

5. Polypeptid, umfassend die Peptidsequenz SEQ ID Nr. 7.

6. Nucleotidsequenz, die ein Polypeptid nach einem der Ansprüche 1 bis 5 codiert.

7. Sequenz nach Anspruch 6, **dadurch gekennzeichnet, dass** sie ausgewählt ist aus:
(a) der Nucleotidsequenz SEQ ID Nr. 1 oder ihrem komplementären Strang,
(b) der ganzen Sequenz, die mit einer Sequenz (a) hybridisiert und ein Polypeptid nach einem der Ansprüche 1 bis 5 codiert, und
(c) den von Sequenzen (a) und (b) abgeleiteten Sequenzen zur Degeneration des genetischen Codes.

8. Sequenz nach Anspruch 7, **dadurch gekennzeichnet, dass** sie die Nucleotidsequenz SEQ ID Nr. 7 umfasst.

9. Sequenz nach Anspruch 7, **dadurch gekennzeichnet, dass** sie ausgewählt ist aus den genomischen Sequenzen, cDNA, RNA, den Hybridsequenzen oder den synthetischen oder halbsynthetischen Sequenzen.

10. Sequenz nach einem der Ansprüche 6 bis 9, **dadurch gekennzeichnet, dass** der das Polypeptid codierende Teil unter die Kontrolle von Signalen gestellt ist, die seine Expression in einer Wirtszelle erlauben.

11. Antisense-Oligonucleotid, das fähig ist, die Herstellung des Polypeptids nach einem der Ansprüche 1 bis 5 zumindest teilweise zu hemmen, **dadurch gekennzeichnet, dass** es aus einer Nucleotidsequenz nach Anspruch 7 besteht.

12. Nucleotidsonde, die fähig ist, mit einer Sequenz nach Anspruch 7 oder entsprechender mRNA zu hybridisieren, **dadurch gekennzeichnet, dass** sie die Nucleotidsequenz SEQ ID Nr. 1, die Nucleotidsequenz SEQ ID Nr. 7 oder ihre komplementären Stränge umfasst.

13. Nucleotidsonde, die fähig ist, mit einer Sequenz nach Anspruch 7 oder entsprechender mRNA zu hybridisieren, **dadurch gekennzeichnet, dass** sie ausgewählt ist aus den Sequenzen SEQ ID Nr. 5, 6, 8-11.

14. In-vitro-Verwendung einer Sonde nach Anspruch 12 oder 13 als Mittel zur Erkennung der Expression eines Polypeptids mit serotoninerger 5HT5a-Rezeptoraktivität; oder zum Nachweis genetischer Anormalitäten (schlechtes Spleissen, Polymorphismus, Punktmutationen, etc); oder zur Identifizierung von neurologischen, kardiovaskulären oder psychiatrischen Erkrankungen, wie jene, die mit den 5HT5a-Rezeptoren in Verbindung stehen; oder weiterhin zum Nachweis und zur Isolierung von homologen Nucleinsäuresequenzen, die das 5HT5a-Polypeptid codieren.

15. Expressionsvektor, **dadurch gekennzeichnet, dass** er eine Nucleotidsequenz nach einem der Ansprüche 6 bis 10 enthält.

16. Rekombinante Zelle, die fähig ist, auf ihrer Oberfläche ein Polypeptid nach einem der Ansprüche 1 bis 5 zu exprimieren, **dadurch gekennzeichnet, dass** sie einen Expressionsvektor nach Anspruch 15 enthält.

17. Zelle nach Anspruch 16, **dadurch gekennzeichnet, dass** sie ausgewählt ist aus den eukaryontischen oder prokaryontischen Zellen.

18. Verfahren zum Nachweis und/oder zur Isolierung von Liganden von wie in den Ansprüchen 1 bis 5 definierten Polypeptiden, **dadurch gekennzeichnet, dass** die folgenden Schritte durchgeführt werden:
- Inkontaktbringen eines Moleküls oder eines Gemischs, das verschiedene Moleküle enthält, die gegebenenfalls nicht identifiziert sind, mit einer rekombinanten Zelle nach Anspruch 16, die auf ihrer Oberfläche ein wie in einem der Ansprüche 1 bis 5 definiertes Polypeptid exprimient, unter Bedingungen, die die Wechselwirkung zwischen dem Polypeptid und dem Molekül erlauben, im Falle, dass es eine Affinität für das Polypeptids besitzt, und,
- Nachweis und/oder Isolierung der mit dem Polypeptid verbundenen Moleküle.

19. Verfahren nach Anspruch 18 zum Nachweis und/oder zur Isolierung von Agonisten oder Antagonisten von Serotinin.

20. Verfahren zum Nachweis und/oder zur Isolierung von Modulatoren der wie in den Ansprüchen 1 bis 5 definierten Polypeptide, **dadurch gekennzeichnet, dass** die folgenden Schritte durchgeführt werden:
- Inkontaktbringen eines Moleküls oder eines Gemischs, das verschiedene Moleküle enthält, die gegebenenfalls nicht identifiziert sind, mit einer rekombinanten Zelle nach Anspruch 16, die auf ihrer Oberfläche ein wie in einem der Ansprüche 1 bis 5 definiertes Polypeptid exprimiert, in Anwesenheit von 5HT, unter Bedingungen, die die Wechselwirkung zwischen dem Polypeptid und 5HT erlauben, und
- Nachweis und/oder Isolierung der Moleküle; die fähig sind, die Aktivität des 5HT auf dem Polypeptid zu modulieren.

## Claims

1. A polypeptide comprising the peptide sequence SEQ ID No. 1.

2. A polypeptide according to claim 1, **characterized in that** it has the capacity to bind serotonin.

3. A polypeptide according to claim 2, **characterized in that** it has serotonin receptor activity.

4. A polypeptide according to one of claims 1 to 3, **characterized in that** it can be recognized by antibodies which recognize the peptide sequence SEQ ID No. 1.

5. A polypeptique comprising the sequence SEQ ID No. 7.

6. A nucleotide sequence coding for a polypeptide according to one of claims 1 to 5.

7. A sequence according to claim 6, **characterized in that** it is selected from:
(a) the nucleotide sequence SEQ ID No. 1 or its complementary strand,
(b) any sequence hybridizing with a sequence (a) and coding for a polypeptide according to one of claims 1 to 5, and
(c) the sequences derived from the sequences (a) and (b) as a result of the degeneracy of the genetic code.

8. A sequence according to claim 7, **characterized in that** it comprises the nucleotide sequence SEQ ID No. 7.

9. A sequence according to claim 7, **characterized in that** it is selected from genomic, cDNA or RNA sequences, hybrid sequences, synthetic and semisynthetic sequences.

10. A sequence according to one of claims 6 to 9, **characterized in that** the part coding for the said polypeptide is placed under the control of signals permitting its expression in a cell host.

11. An antisense oligonucleotide capable of at least partially inhibiting the production of polypeptides according to one of claims 1 to 5, **characterized in that** it consists of a nucleotide sequence according to claim 7.

12. A nucleotide probe capable of hybridizing with a sequence according to claim 7 or with the corresponding mRNA, **characterized in that** it comprises the nucleotide sequence SEQ ID No. 1, the nucleotide sequence SEQ ID No. 7 or their complementary strands.

13. A nucleotide probe capable of hybridising with a sequence according to claim 7 or with the corresponding mRNA, **characterized in that** it is selected from the sequences SEQ ID No. 5, 6, 8-11.

14. *In vitro* use of a probe according to claim 12 or 13 as an agent for detecting the expression of a 5HT5a serotonin receptor, for demonstrating genetic abnormalities (incorrect splicing polymorphism, point mutations, and the like), for identifying neurological, cardiovascular or psychiatric disorders as being associated with 5HT5a receptors, or for demonstrating and isolating homologous nucleic acid sequences coding for 5HT5a polypeptides.

15. Expression vector, **characterized in that** it contains a nucleotide sequence according to one of claims 6 to 10.

16. A recombinant cell capable of expressing at its surface a polypeptide according to one of claims 1 to 5, **characterized in that** it contains an expression vector according to claim 15.

17. A recombinant cell according to claim 16, **characterized in that** it is selected from eukaryotic cells and prokaryotic cells.

18. A method for demonstrating and/or isolating ligands of the polypeptides as defined in claims 1 to 5, **characterized in that** the following steps are carried out:
- a molecule or a mixture containing different molecules which are possibly unidentified, is contacted with a recombinant cell according to claim 16 expressing at its surface a polypeptide as defined in claims 1 to 5, under conditions permitting interaction between the said polypeptide and the said molecule, and
- the molecules bound to the said polypeptide are detected and/or isolated.

19. A method according to claim 18 for demonstrating and/or isolating agonists or antagonists of serotonin.

20. A method for demonstrating and/or isolating modulators of the polypeptides as defined in claims 1 to 5, **characterized in that** the following steps are carried out:
- a molecule or a mixture containing different molecules which are possibly unidentified, is contacted with a recombinant cell according to claim 16 expressing at its surface a polypeptide as defined in claims 1 to 5, in the presence of 5HT, under conditions permitting interaction between the said polypeptide and 5HT, and
- the molecules capable of modulating the activity of 5HT with respect to the said polypeptide are detected and/or isolated.
